# EUROPEAN PATENT SPECIFICATION

(11) **EP 3 285 817 B1**
(45) Date of publication and mention of the grant of the patent: **12.06.2019**
(21) Application number: 16722521.8
(22) Date of filing: 11.04.2016
(51) Int. Cl.: A61L 27/16, A61L 27/50, A61L 27/54, C08K 5/00, C08K 5/1545, C08K 5/20, C08K 5/40

(54) **COMPOSITION FOR STABILIZATION OF POLYETHYLENE, POLYETHYLENE, METHOD OF PREPARATION THEREOF, POLYETHYLENE IMPLANTS**
ZUSAMMENSETZUNG ZUR STABILISIERUNG VON POLYETHYLEN, POLYETHYLEN, VERFAHREN ZUR HERSTELLUNG DAVON, POLYETHYLENIMPLANTATE
COMPOSITION DE STABILISATION DE POLYÉTHYLÈNE, POLYÉTHYLÈNE, PROCÉDÉ DE PRÉPARATION DE CE DERNIER, IMPLANTS DE POLYÉTHYLÈNE

(30) Priority: 21.04.2015 CZ 20150267
(43) Date of publication of application: 28.02.2018
(73) Proprietor: Ustav Makromolekularni Chemie AV CR, v.v.i., 162 06 Praha 6 (CZ)
(72) Inventor: KRULIS, Zdenek, 15500 Praha 5 (CZ); SLOUF, Miroslav, 10700 Praha 15 (CZ); BENES, Hynek, 19800 Praha 9 (CZ); KOVAROVA, Jana, 12000 Praha 2 (CZ); MICHALKOVA, Danuse, 15000 Praha 5 (CZ); NEVORALOVA, Martina, 27341 Brandysek (CZ)
(74) Representative: Hartvichova, Katerina
(86) International application number: PCT/CZ2016/050010
(87) International publication number: WO 2016/169536

(56) References cited:
- WO-A1-2007/019874
- WO-A2-2010/129514
- US-A1- 2006 223 905
- DATABASE WPI Week 201523 Thomson Scientific, London, GB; AN 2015-183742 XP002758787, & CN 104 288 162 A (ZHENGZHOU BORY ANIMAL PHARM CO LTD) 21 January 2015 (2015-01-21)

## Description

### Field of Art

The invention relates to stabilizing composition, and to polyethylene stabilized by this composition against oxidative degradation, said polyethylene being useful for production of implants used in human or veterinary medicine.

### Background Art

The specific application of polyethylene (especially ultra-high-molecular-weight polyethylene, UHMWPE) in joint replacements requires that this material is well tolerated by the organism, resistant to aggressive body fluids, and that it maintains the necessary physical and mechanical qualities over the whole expected lifetime of said replacement.

Practice has shown that some applications require that the UHMWPE material is cross-linked by means of high-energy irradiation, in order to achieve the optimum combination of the properties of said material, in particular increase of its wear resistance. One of the major factors affecting long-term utility of the thus modified materials is the lifetime of the replacement in given conditions, in particular the stability against oxidative degradation.

Long-term oxidative stability of UHMWPE is one of the basic prerequisites for achieving the desired lifetime of joint replacements. Oxidation results in hardening and embrittlement of polyethylene in the depth of several tenths of millimeter up to a millimeter. Even though the aging of the UHMWPE joint replacements was thoroughly studied, the exact mechanism of UHMWPE oxidation in human body has not been elucidated completely.

The UHMWPE oxidation is a complex succession of cascade reactions, the course of which is not exactly known. Enzymatic redox reactions (caused by the action of dehydrogenase and oxidase) occurring among the cells lead to the formation of significant amounts of free radicals of varying stability. The presence of the polyethylene molecule in close proximity to the cell leads to interference with these reactions, e.g., to activation of hydrogen transfer from a chain tertiary carbon, resulting in redox degradation of polyethylene taking place via radical mechanism.

Effective stabilizers for polyolefins were developed and are commercially available.

However, they are not suitable for medical applications as joint replacements, according to hygienic-toxicologic criteria. Hence, their alternatives were sought for. Doležel and Adamírová (CZ 221 404, 1982) were the first to describe increasing oxidative stability of polyethylene by incorporation of tocoferols (vitamin E). Besides vitamin E, further biologically acceptable substances were provided as polyethylene antioxidants. Hahn has described the antioxidative effect of carotenoids, in particular beta-carotene, to UHMWPE (US 5,827,904). Later on, several processes improving the wear resistance of UHMWPE were described, based on the combination of vitamin E addition and radiation cross-linking. Some documents disclose stirring of vitamin E into pulverized UHMWPE, its subsequent processing and radiation cross-linking (US 6,277,390, US 6,448,315), yet other documents describe diffusion of vitamin E directly into products made of UHMWPE, usually at an elevated temperature (WO 2004064618, WO 20055110276, CA 256129). The drawback of the former technique is a lower achievable cross-linking density of UHMWPE in comparison to a non-stabilized material. The latter technique, based on diffusion, does not provide any certainty as to the concentration of the incorporated antioxidant.

Improvement of the utility properties of UHMWPE for medical applications, designed to remove these drawbacks, is based on stabilization of the material by a combination of selected flavonoids with selected amino acids before the radiation cross-linking of said material (US 8,586,667).

WO 2010/129514 describes the preparation of crosslinked oxidation resistant UHMWPE suitable for use in medical prostheses, wherein the UHMWPE resin is combined with two additives and crosslinked. The additives may include phenolic antioxidants and hindered amines.

The present invention aims at providing polyethylene, in particular UHMWPE, stabilized by biologically acceptable substances.

The solution provided by the present invention uses substances that are even used a medicaments or food supplements.

### Disclosure of the Invention

The invention is based on the surprising fact that combination of tocoferols and/or tocotrienols, optionally with addition of tetraethylthiuramdisulfide, and of tetracycline antibiotic shows a significant synergic antioxidation, i.e., stabilizing, effect in polyethylene. Furthermore, it was found that the stabilizing system based on this synergic mixture does not substantially decrease cross-linking effect of gamma-irradiation or accelerated electrons irradiation. Most significant synergic effect was found for the mixture of alpha-tocoferol and tetracycline. Additionally, it was discovered that the synergic antioxidant effect of the combination of tocoferols and/or tocotrienols with tetracycline antibiotic to polyethylene is further increased by the presence of tetraethylthiuramdisulfide.

Tocoferol and/or tocotrienol alone with tetraethylthiuramdisulfide, or tetracycline alone with tetraethylthiuramdisulfide do not show this synergic effect.

Object of the present invention is thus a composition for stabilization of polyethylene, said composition consisting of a first component selected from the group consisting of tocoferols, tocotrienols and mixtures thereof, and of a second component which is at least one tetracycline-type antibiotic, wherein the first component further contains tetraethylthiuramdisulfide in mass ratio of 1:3 to 1:1, relative to the total tocoferols and/or tocotrienols.

In a preferred embodiment, the second component is selected from the group consisting of tetracycline, oxytetracycline and chlorotetracycline.

Preferably, the ratio of the first and second components is in the range of 1:5 to 5:1.

The stabilizing system based on tocoferols and/or tocotrienols and a tetracycline antibiotic enables the preparation of polyethylene cross-linked by ionization radiation, said polyethylene having an increased resilience and wear resistance, as well as increased resistance to oxidative degradation. Materials possessing these properties in combination are suitable in particular for production of sliding parts of total joint replacements.

It was discovered that the cross-linking effect of the ionization radiation on polyethylene stabilized by the combination of tocoferols and/or tocotrienols and tetracycline antibiotic is higher in the presence of sterically hindered amines based on 2,2,6,6-tetramethyl piperidine derivatives having molar weight of at least 480 g/mol and at most 4100 g/mol, whereas the oxidative stability of the radiation-cross-linked material is equal or higher than that of polyethylene stabilized only by the combination of tocoferols and/or tocotrienols and tetracycline antibiotic.

Thus, in another preferred embodiment, the composition for stabilization of polyethylene further comprises 20 to 80 wt. % of the hindered amine light stabilizer (HALS), relative to the total weight of the compostion. HALS are typically sterically hindered polymeric or oligomeric amines containing tetramethyl piperidine units, and/or amino- and/or C1-C10 alkylamino-substituted 1,3,5-triazine units, whereas the polymeric or oligomeric amine has molar weight of 480 g/mol to 4500 g/mol. HALS are well known to the person skilled in the art.

The invention further includes the use of compositions consisting of a first component selected from the group consisting of tocoferols, tocotrienols and mixtures thereof, and of a second component which is at least one tetracycline-type antibiotic, optionally further containing other components as described herein above for stabilizing polyethylene.

Object of the present invention is also polyethylene having weight average molecular weight of 5.10⁵ g/mol to 6.10⁶ g/mol comprising the stabilizing composition of this invention, said composition consisting of a first component selected from the group consisting of tocoferols, tocotrienols and mixtures thereof, and of a second component which is at least one tetracycline-type antibiotic, and optionally further containing other components as described herein above. Preferably, polyethylene contains at least 200 ppm (by weight) of the stabilizing composition of the present invention.

In one preferred embodiment, the polyethylene is ultra-high-molecular-weight polyethylene (UHMWPE).

Furthermore, object of the present invention is a method of preparation of stabilized polyethylene having average molar weight lower than 1.10⁶ g/mol, wherein the components of the composition are incorporated into polyethylene by melt stirring.

Yet furthermore, object of the present invention is a method of preparation of stabilized polyethylene having average molar weight 1.10⁶ g/mol or higher, which cannot be processed using technologies appropriate for plastics. In this case, the components of the composition are mixed with pulverized polyethylene to form a dry mixture which is then subjected to pressing or intrusion process, i.e., by extrusion from piston-extruding apparatus at a temperature higher than the melting temperature of the crystalline phase of the used polyethylene.

Object of the present invention is also a method of preparation of stabilized radiation-cross-linked polyethylene, wherein in the first step, the components of the stabilizing composition are incorporated to polyethylene by the above-described methods, and subsequently, the polyethylene is cross-linked by action of ionization radiation in inert atmosphere in a irradiation dose of 50 kGy to 200 kGy. The radiation cross-linking of the polyethylene and stabilizers is preferably carried out using γ-irradiation with radiation dose rate of 0,7 kGy/h to 10 kGy/h, or using accelerated electrons irradiation with radiation dose rate of 1 MGy/h to 10 MGy/h.

An advantage of the stabilizing composition according to the present invention is that the basic components, i.e., tetracycline antibiotics and tocoferols and/or tocotrienols are established medicaments or are essential components of human nutrition (vitamin E is a mixture of tocoferols and tocotrienols), and their application as stabilizers of polyethylene useful for production of implants minimizes risk of undesirable side effect to human or animal organism. Transport of the antibiotic from the implant material to surrounding tissues or interstitial fluid is practically impossible due to strongly hydrophobic properties of polyethylene.

### Examples of carrying out the Invention

### Example 1

High density polyethylene having an average molecular weight *M*_{w} = 900 000 g/mol and a stabilizer were melt mixed in laboratory kneader at the temperature of 190 °C and rpm 60 min⁻¹ to form mixtures containing 1500 ppm of the stabilizer. In order to compare the stabilizing effects, the polyethylene was stabilized by α-tocoferol (mixture A, which is a reference-example), combination of α-tocoferol with tetracycline in mass ratio 2:1 (mixture B), combination of α-tocoferol with tetracycline in mass ratio 1:1 (mixture C) and combination of α-tocoferol with tetracycline in mass ratio 1:2 (mixture D). Thermooxidation stability of the materials was determined as the temperature of oxidative reaction onset from calorimetric measurements (differential scanning calorimetry, DSC). The results of the measurements are shown in table 1.

### Example 2

High density polyethylene having an average molecular weight *M*_{w} = 900 000 g/mol and a stabilizer were melt mixed in laboratory kneader at the temperature of 190 °C and rpm 60 min⁻¹ to form mixtures containing 1500 ppm of the stabilizer. Polyethylene was stabilized by combination of vitamin E with tetracycline in mass ratio 1:1 (mixture E) and 1:2 (mixture F). Thermooxidation stability of the materials was determined as the temperature of oxidative reaction onset from calorimetric measurements by the same method as in Example 1. The results of the measurements are shown in table 1.

### Example 3

High density polyethylene having an average molecular weight *M*_{w} = 900 000 g/mol and a stabilizer were melt mixed in laboratory kneader at the temperature of 190 °C and rpm 60 min⁻¹ to form mixtures containing 1500 ppm of the stabilizer, said stabilizer being a composition consisting of vitamin E, tetracycline and tetraethylthiuramdisulfide in mass ratio 1:1:1 (mixture G). Thermooxidation stability of the materials was determined as the temperature of oxidative reaction onset from calorimetric measurements by the same method as in Example 1. The results of the measurements are shown in table 1.

### Example 4

Pulverized ultra-high-molecular-weight polyethylene having an average molecular weight *M*_{w} = 3 000 000 g/mol and a stabilizer were mixed to form dry mixtures containing 1500 ppm of the stabilizer. In order to compare the stabilizing effects, the polyethylene was stabilized by α-tocoferol (mixture H, which is a reference-example), combination of α-tocoferol with tetracycline in mass ratio 2:1 (mixture I), combination of α-tocoferol with tetracycline in mass ratio 1:1 (mixture K) and combination of α-tocoferol with tetracycline in mass ratio 1:2 (mixture L). The thus prepared dry mixtures were processed by pressing at the temperature of 190 °C into plates having the thickness of 4 mm. Thermooxidation stability of the materials was determined as the temperature of oxidative reaction onset by differential scanning calorimetry (DSC). The results of the measurements are shown in table 2.

### Example 5

Pulverized ultra-high-molecular-weight polyethylene having an average molecular weight *M*_{w} = 3 000 000 g/mol and a stabilizer were mixed to form dry mixtures containing 1500 ppm of the stabilizer. In order to compare the stabilizing effects, the polyethylene was stabilized by α-tocoferol (mixture M, which is a reference-example), combination of α-tocoferol with tetracycline in mass ratio 1:1 (mixture N) and combination of α-tocoferol with tetracycline in mass ratio 1:2 (mixture O). The thus prepared dry mixtures were processed by pressing at the temperature of 190 °C into plates having the thickness of 4 mm. The plates were irradiated by γ-radiation from the ⁶⁰Co source in nitrogen atmosphere, using an irradiation dose of 104 kGy at dose rate of the γ-radiation being 1.05 kGy/h. After irradiation, the plates were remelted in a mold under nitrogen atmosphere, at 150 °C for 10 min. Thermooxidation stability of the materials was determined as the temperature of oxidative reaction onset by differential scanning calorimetry (DSC). The results of the measurements are shown in table 2.

### Example 6

Pulverized ultra-high-molecular-weight polyethylene having an average molecular weight *M*_{w} = 3 000 000 g/mol and a stabilizer were mixed to form dry mixtures containing 1500 ppm of the stabilizer. In order to compare the stabilizing effects, the polyethylene was stabilized by α-tocoferol (mixture P, which is a reference-example), combination of α-tocoferol with tetracycline in mass ratio 1:1 (mixture Q) and combination of α-tocoferol with tetracycline in mass ratio 1:2 (mixture R). The thus prepared dry mixtures were processed by pressing at the temperature of 190 °C into plates having the thickness of 4 mm. The plates were irradiated by accelerated electrons, using an irradiation dose of 100 kGy at dose rate 1.0 kGy/h. After irradiation, the plates were remelted under nitrogen atmosphere, at 150 °C for 10 min. Thermooxidation stability of the materials was determined as the temperature of oxidative reaction onset by differential scanning calorimetry (DSC). The results of the measurements are shown in table 3.

### Example 7

Pulverized ultra-high-molecular-weight polyethylene having an average molecular weight *M*_{w} = 3 000 000 g/mol and a stabilizer were mixed to form dry mixtures containing 1500 ppm of the stabilizer consisting of α-tocoferol with tetracycline and light stabilizer (HALS) based on tetramethylpiperidine derivative in mass ratio 1:1:1. Mixture S contains oligomeric piperidine derivative poly[[6-[(1,1,3,3-tetramethylbutyl)amino]-1,3,5-triazine-2,4-diyl][(2,2,6,6-tetramethyl-4-piperidinyl)imino]-1,6-hexanediyl[(2,2,6,6-tetramethyl-4-piperidinyl)imino]]) having molar weight 2600 g/mol, commercially available under the trade name Chimassorb 944, mixture T contains piperidine derivative 1,3,5-triazine-2,4,6-triamine, N,N"'-1,2-ethanediylbis[N-[3-[[4,6-bis[butyl(1,2,2,6,6-pentamethyl-4-piperidinyl)amino]-1,3,5-triazin-2-yl]amino]propyl]-N',N"-dibutyl-N',N"-bis(1,2,2,6,6-pentamethyl-4-piperidinyl) commercialy available under the trade name Chimassorb 119, mixture U contains oligomeric piperidine derivative based on polymeric reaction product of 2,4,6-trichloro-1,3,5-triazine with mixture of N-butyl-1-butanamine and N-butyl-2,2,6,6-tetramethyl-4-piperidinamine having the average molecular weight M_{w} = 4050 g/mol, commercially available under the trade name Tinuvin NOR 371. The thus prepared dry mixtures were processed by pressing at the temperature of 190 °C into plates having the thickness of 4 mm. The plates were irradiated by accelerated electrons in nitrogen atmosphere, using an irradiation dose of 100 kGy at dose rate 1.0 MGy/h. After irradiation, the plates were remelted under nitrogen atmosphere, at 150 °C for 10 min. Thermooxidation stability of the materials was determined as the temperature of oxidative reaction onset by differential scanning calorimetry (DSC). The results of the measurements are shown in table 3.

**Table 1: Temperature of oxidation onset (Tₒₙₛₑₜ) for ultra-high-molecular-weight polyethylene, non-stabilized and stabilized according to Examples 1, 2 and 3**

| Stabilizer components; ppm | Mixture/Example | | | | | | | |
|---|---|---|---|---|---|---|---|---|
| | Non-stabilized PE | A/1 | B/1 | C/1 | D/1 | E/2 | F/2 | G/3 |
| α-tocoferol | - | 1500 | 1000 | 750 | 500 | - | - | - |
| Vitamin E | - | - | - | - | - | 750 | 500 | 500 |
| Tetracyclin | - | - | 500 | 750 | 1000 | 750 | 1000 | 500 |
| Tetraethylthiuramdisulfid | - | - | - | - | - | - | - | 500 |
| *T*ₒₙₛₑₜ; °C | 204 | 257 | 270 | 271 | 273 | 270 | 271 | 274 |

**Table 2: Temperature of oxidation onset (Tₒₙₛₑₜ) for ultra-high-molecular-weight polyethylene modified by γ-irradiation, non-stabilized and stabilized according to Examples 4 and 5**

| Stabilizer components; ppm | Mixture/Example | | | | | | | | |
|---|---|---|---|---|---|---|---|---|---|
| | Non-stabilized UHMWPE | H/4 | 1/4 | K/4 | L/4 | Non-stabilized UHMWPE | M/5 | N/5 | O/5 |
| α-tocoferol | - | 1500 | 1000 | 750 | 500 | - | 1500 | 750 | 500 |
| Tetracyclin | - | - | 500 | 750 | 1000 | - | - | 750 | 1000 |
| Irradiation dose of γ-radiation, kGy | - | - | - | - | - | 104 | 104 | 104 | 104 |
| *T*ₒₙₛₑₜ; °C | 211 | 264 | 276 | 275 | 278 | 198 | 205 | 215 | 217 |

**Table 3: Temperature of oxidation onset (Tₒₙₛₑₜ) for ultra-high-molecular-weight polyethylene modified by accelerated electron irradiation (irradiation dose 100 kGy), non-stabilized and stabilized according to Examples 6 and 7**

| Stabilizer components; ppm | Mixture/Example | | | | | | |
|---|---|---|---|---|---|---|---|
| | Non-stabilized UHMWPE | P/6 | Q/6 | R/6 | S/7 | T/7 | U/7 |
| α-tocoferol | - | 1500 | 750 | 500 | 500 | 500 | 500 |
| Tetracyclin | - | - | 750 | 1000 | 500 | 500 | 500 |
| Chimassorb 944 | - | - | - | - | 500 | - | - |
| Chimassorb 119 | - | - | - | - | - | 500 | - |
| Tinuvin NOR 371 | - | - | - | - | - | - | 500 |
| *T*ₒₙₛₑₜ; °C | 199 | 204 | 215 | 216 | 226 | 225 | 225 |

### Industrial Applicability

Stabilizer (composition for stabilization) of the present invention is particularly useful for stabilization of polyethylene for medical applications, and the polyethylene stabilized according to the invention is particularly suitable for production of implants used in human and veterinary medicine.

## Claims

1. Composition for stabilization of polyethylene, **characterized in that** it consists from a first component selected from the group consisting of tocoferols, tocotrienols and mixtures thereof, and from a second component which is at least one tetracycline antibiotic, wherein the first component further contains tetraethylthiuramdisulfide in a mass ratio relative to total tocoferols and tocotrienols 1:3 to 1:1.

2. The composition according to claim 1, wherein the second component is selected from the group consisting of tetracycline, oxytetracycline and chlorotetracycline.

3. The composition according to any one of the preceding claims, wherein the mass ratio of the first and second component is from 1:5 to 5:1.

4. The composition according to any one of the preceding claims, which further comprises 20 to 80 wt. % of hindered amine light stabilizer.

5. Use of a composition consisting from a first component selected from the group consisting of tocoferols, tocotrienols and mixtures thereof, and from a second component which is at least one tetracycline antibiotic for stabilizing polyethylene, preferably for stabilizing polyethylene having average molar weight of 5.10⁵ g/mol to 6.10⁶ g/mol.

6. Polyethylene having average molar weight of 5.10⁵ g/mol to 6.10⁶ g/mol, **characterized in that** it contains a composition consisting from a first component selected from the group consisting of tocoferols, tocotrienols and mixtures thereof, and from a second component which is at least one tetracycline antibiotic , preferably in an amount of at least 200 ppm of the composition.

7. Use according to claim 5 or polyethylene according to claim 6, wherein the first component further contains tetraethylthiuramdisulfide in a mass ratio relative to total tocoferols and tocotrienols 1:3 to 1:1.

8. Use according to claim 5 or polyethylene according to claim 6, wherein the composition further comprises 20 to 80 wt. % of hindered amine light stabilizer.

9. Use according to claim 5 or polyethylene according to claim 6, wherein the second component is selected from the group consisting of tetracycline, oxytetracycline and chlorotetracycline.

10. Use according to claim 5 or polyethylene according to claim 6, wherein the mass ratio of the first and second component is from 1:5 to 5:1.

11. A method of preparation of the polyethylene according to any one of claims 6 to 10, having average molar weight lower than 1.10⁶ g/mol, containing a composition that consists from a first component selected from the group consisting of tocoferols, tocotrienols and mixtures thereof, and from a second component which is at least one tetracycline antibiotic, or the composition of any one of claims 1 to 4, **characterized in that** the components of the composition are incorporated into polyethylene by melt mixing.

12. A method of preparation of the polyethylene according to any one of claims 6 to 10, having average molar weight 1.10⁶ g/mol or higher, containing a composition that consists from a first component selected from the group consisting of tocoferols, tocotrienols and mixtures thereof, and from a second component which is at least one tetracycline antibiotic, or the composition of any one of claims 1 to 4, **characterized in that** the components of the composition are mixed with pulverized polyethylene to form a dry mixture which is then subjected to pressing or intrusion process.

13. The method according to claim 11 or 12, wherein the polyethylene is subsequently cross-linked by action of ionization radiation in inert atmosphere in an irradiation dose of 50 kGy to 200 kGy, wherein the radiation cross-linking of the polyethylene and stabilizers is preferably carried out using γ-irradiation with dose rate of 0.7 kGy/h to 10 kGy/h, or using accelerated electrons irradiation with dose rate of 1 MGy/h to 10 MGy/h.

14. Implant for use in human or veterinary medicine, **characterized in that** it contains polyethylene according to any one of claims 6 to 10.

15. A method for stabilization of polyethylene, **characterized in that** the composition according to any one of claims 1 to 4 is added to the polyethylene.

## Patentansprüche

1. Zusammensetzung zur Stabilisierung von Polyethylen, **dadurch gekennzeichnet, dass** sie aus einer ersten Komponente ausgewählt aus der Gruppe bestehend aus Tocoferolen, Tocotrienolen und Mischungen davon und aus einer zweiten Komponente, die mindestens ein Tetracyclin-Antibiotikum ist, besteht, wobei die erste Komponente weiterhin Tetraethylthiuramdisulfid in einem Massenverhältnis relativ zu den gesamten Tocoferolen und Tocotrienolen 1:3 bis 1:1 enthält.

2. Zusammensetzung nach Anspruch 1, wobei die zweite Komponente ausgewählt ist aus der Gruppe bestehend aus Tetracyclin, Oxytetracyclin und Chlortetracyclin.

3. Zusammensetzung nach einem der vorhergehenden Ansprüche, wobei das Massenverhältnis der ersten und der zweiten Komponente 1:5 bis 5:1 beträgt.

4. Zusammensetzung nach einem der vorhergehenden Ansprüche, die ferner 20 bis 80 Gew. % des gehinderten Amin-Lichtstabilisators umfasst.

5. Verwendung einer Zusammensetzung, bestehend aus einer ersten Komponente, ausgewählt aus der Gruppe bestehend aus Tocoferolen, Tocotrienolen und Mischungen davon, und aus einer zweiten Komponente, die mindestens ein Tetracyclin-Antibiotikum ist, zum Stabilisieren von Polyethylen, vorzugsweise zum Stabilisieren von Polyethylen mit einem durchschnittlichen Molgewicht von 5.10⁵ g/mol bis 6.10⁶ g/mol.

6. Polyethylen mit einem durchschnittlichen Molgewicht von 5.10⁵ g/mol bis 6.10⁶ g/mol, **dadurch gekennzeichnet, dass** es eine Zusammensetzung enthält, die besteht aus einer ersten Komponente ausgewählt aus der Gruppe bestehend aus Tocoferolen, Tocotrienolen und Mischungen davon und einer zweiten Komponente, die mindestens ein Tetracyclinantibiotikum ist, vorzugsweise in einer Menge von mindestens 200 ppm der Zusammensetzung.

7. Verwendung nach Anspruch 5 oder Polyethylen nach Anspruch 6, wobei die erste Komponente weiterhin Tetraethylthiuramdisulfid in einem Massenverhältnis relativ zu den gesamten Tocoferolen und Tocotrienolen 1:3 bis 1:1 enthält.

8. Verwendung nach Anspruch 5 oder Polyethylen nach Anspruch 6, wobei die Zusammensetzung weiterhin 20 bis 80 Gew. % des gehinderten Amin-Lichtstabilisators umfasst.

9. Verwendung nach Anspruch 5 oder Polyethylen nach Anspruch 6, wobei die zweite Komponente ausgewählt ist aus der Gruppe bestehend aus Tetracyclin, Oxytetracyclin und Chlortetracyclin.

10. Verwendung nach Anspruch 5 oder Polyethylen nach Anspruch 6, wobei das Massenverhältnis der ersten und der zweiten Komponente 1:5 bis 5:1 beträgt.

11. Verfahren zur Herstellung des Polyethylens nach einem der Ansprüche 6 bis 10 mit einem durchschnittlichen Molgewicht von weniger als 1.10⁶ g/mol, enthaltend eine Zusammensetzung bestehend aus einer ersten Komponente, die aus der Gruppe bestehend aus Tocoferolen, Tocotrienolen und Mischungen davon ausgewählt ist, und aus einer zweiten Komponente, die mindestens ein Tetracyclinantibiotikum ist, oder enthaltend der Zusammensetzung nach einem der Ansprüche 1 bis 4, **dadurch gekennzeichnet, dass** die Komponenten der Zusammensetzung durch Schmelzmischen in Polyethylen eingearbeitet werden.

12. Verfahren zur Herstellung des Polyethylens nach einem der Ansprüche 6 bis 10 mit einem durchschnittlichen Molgewicht von 1.10⁶ g/mol oder mehr, enthaltend eine Zusammensetzung bestehend aus einer ersten Komponente, die aus der Gruppe bestehend aus Tocoferolen, Tocotrienolen und Mischungen davon ausgewählt ist, und aus einer zweiten Komponente, die mindestens ein Tetracyclinantibiotikum ist, oder enthaltend der Zusammensetzung nach einem der Ansprüche 1 bis 4, **dadurch gekennzeichnet, dass** die Komponenten der Zusammensetzung mit pulverisiertem Polyethylen gemischt werden, um eine trockene Mischung zu bilden, die dann unterworfen wird Press- oder Einbruchsvorgang.

13. Verfahren nach Anspruch 11 oder 12, bei dem das Polyethylen anschließend durch Einwirkung von Ionisationsstrahlung in inerter Atmosphäre in einer Bestrahlungsdosis von 50 kGy bis 200 kGy vernetzt wird, wobei die Strahlungsvernetzung des Polyethylens und der Stabilisatoren ist vorzugsweise durchgeführt unter Verwendung von γ-Bestrahlung mit einer Dosisrate von 0,7 kGy/h bis 10 kGy/h oder unter Verwendung einer Bestrahlung mit beschleunigten Elektronen mit einer Dosisrate von 1 MGy/h bis 10 MGy/h.

14. Implantat zur Verwendung in der Human- oder Veterinärmedizin, **dadurch gekennzeichnet, dass** es Polyethylen nach einem der Ansprüche 6 bis 10 enthält.

15. Verfahren zur Stabilisierung von Polyethylen, **dadurch gekennzeichnet, dass** die Zusammensetzung nach einem der Ansprüche 1 bis 4 dem Polyethylen zugesetzt wird.

## Revendications

1. Composition pour la stabilisation de polyéthylène, **caractérisée en ce qu'**elle consiste d'un premier composant choisi dans le groupe constitué des tocoférols, des tocotriénols et leurs mélanges, et d'un deuxième composant qui est au moins un antibiotique de la classe des tétracyclines, où le premier composant contient en outre du tétraéthylthiuramdisulfure dans un rapport de masse, par rapport au total des tocoférols et des tocotriénols, de 1:3 à 1:1.

2. Composition selon la revendication 1, dans laquelle le deuxième composant est choisi dans le groupe constitué par la tétracycline, l'oxytétracycline et la chlorotétracycline.

3. Composition selon l'une quelconque des revendications précédentes, dans laquelle le rapport de masse des premier et deuxième composants est de 1:5 à 5:1.

4. Composition selon l'une quelconque des revendications précédentes, qui comprend en outre de 20 à 80 % en poids de stabilisant à la lumière de type amine encombrée.

5. Utilisation d'une composition constituée d'un premier composant choisi dans le groupe constitué par les tocoférols, les tocotriénols et leurs mélanges, et d'un deuxième composant qui est au moins un antibiotique de la classe des tétracyclines, pour la stabilisation du polyéthylène, de préférence pour la stabilisation du polyéthylène ayant un poids molaire moyen de 5.10⁵ g/mol à 6.10⁶ g/mol.

6. Polyéthylène d'un poids molaire moyen de 5.10⁵ g/mol à 6.10⁶ g/mol, **caractérisé en ce qu'**il contient une composition consistant en un premier composant choisi dans le groupe constitué par les tocoférols, les tocotriénols et leurs mélanges, et d'un deuxième composant qui est au moins un antibiotique de la classe des tétracyclines, de préférence en une quantité d'au moins 200 ppm de la composition.

7. Utilisation selon la revendication 5 ou polyéthylène selon la revendication 6, où le premier composant contient en outre du tétraéthylthiuramdisulfure dans un rapport de masse, par rapport au total des tocoférols et des tocotriénols, de 1:3 à 1:1.

8. Utilisation selon la revendication 5 ou polyéthylène selon la revendication 6, où la composition comprend en outre de 20 à 80 % en poids de stabilisant à la lumière de type amine encombrée.

9. Utilisation selon la revendication 5 ou polyéthylène selon la revendication 6, où le deuxième composant est choisi dans le groupe constitué par la tétracycline, l'oxytétracycline et la chlorotétracycline.

10. Utilisation selon la revendication 5 ou polyéthylène selon la revendication 6, où le rapport de masse des premier et deuxième composants est de 1:5 à 5:1.

11. Procédé de préparation du polyéthylène selon l'une quelconque des revendications 6 à 10, ayant un poids molaire moyen inférieur à 1.10⁶ g/mol, contenant une composition qui consiste d'un premier composant choisi dans le groupe constitué des tocoférols, des tocotriénols et leurs mélanges, et d'un deuxième composant qui est au moins un antibiotique de la classe des tétracyclines, ou la composition selon l'une quelconque des revendications 1 à 4, **caractérisé en ce que** les composants de la composition sont incorporés dans du polyéthylène par mélange à l'état fondu.

12. Procédé de préparation du polyéthylène selon l'une quelconque des revendications 6 à 10, ayant un poids molaire moyen égal ou supérieur à 1,10⁶ g/mol, contenant une composition qui consiste d'un premier composant choisi dans le groupe constitué des tocoférols, des tocotriénols et leurs mélanges, et d'un deuxième composant qui est au moins un antibiotique de la classe des tétracyclines, ou la composition selon l'une quelconque des revendications 1 à 4, **caractérisé en ce que** les composants de la composition sont mélangés avec du polyéthylène pulvérisé pour former un mélange sec qui est ensuite soumis à processus de pressage ou d'intrusion.

13. Procédé selon la revendication 11 ou 12, dans lequel le polyéthylène est ensuite réticulé par action d'un rayonnement ionisant dans une atmosphère inerte à une dose d'irradiation de 50 kGy à 200 kGy, dans laquelle la réticulation du polyéthylène et des stabilisants par rayonnement est de préférence réalisée en utilisant une irradiation γ avec un débit de dose de 0,7 kGy/h à 10 kGy/h, ou en utilisant une irradiation aux électrons accélérés avec un débit de dose de 1 MGy/h à 10 MGy/h.

14. Implant destiné à être utilisé en médecine humaine ou vétérinaire, **caractérisé en ce qu'**il contient du polyéthylène selon l'une quelconque des revendications 6 à 10.

15. Procédé de stabilisation du polyéthylène, **caractérisé en ce que** la composition selon l'une quelconque des revendications 1 à 4 est ajoutée au polyéthylène.
